# EUROPEAN PATENT SPECIFICATION

(11) **EP 2 968 035 B1**
(45) Date of publication and mention of the grant of the patent: **11.07.2018**
(21) Application number: 14770206.2
(22) Date of filing: 26.02.2014
(51) Int. Cl.: A61F 13/551, A61F 13/511

(54) **ABSORBENT ARTICLES AND METHODS OF MANUFACTURING THE SAME**
SAUGFÄHIGE ARTIKEL UND VERFAHREN ZUR HERSTELLUNG DAVON
ARTICLES ABSORBANTS ET LEURS PROCÉDÉS DE FABRICATION

(30) Priority: 15.03.2013 US 201313832965
(43) Date of publication of application: 20.01.2016
(73) Proprietor: Medline Industries, Inc., Mundelein, IL 60060 (US)
(72) Inventor: NELSON, Christopher, J., Plymouth, WI 53073 (US); ROVERO, William, Douglasville, GA 30135 (US); LOVE, Daniel, B., Libertyville, IL 60048 (US)
(74) Representative: Molinari, Marinella
(86) International application number: PCT/US2014/018500
(87) International publication number: WO 2014/149446

(56) References cited:
- WO-A1-99/48452
- US-A1- 2003 114 808
- US-A1- 2006 069 370
- US-A1- 2008 286 320
- US-A1- 2012 271 267
- US-B1- 6 315 765
- US-B1- 6 673 984
- US-B2- 6 939 553

## Description

### RELATED APPLICATION

This application claims priority to U.S. Patent Application No. 13/832,965 filed March 15, 2013 entitled "ABSORBENT ARTICLES AND METHODS OF MANUFACTURING THE SAME" which is a continuation-in-part of copending U.S. Patent Application No. 13/276,843 filed October 19, 2010 and entitled "ABSORBENT ARTICLES AND METHODS OF MANUFACTURING THE SAME," which application claims priority to U.S. Provisional Application No. 61/394,758 filed October 19, 2011 and entitled "ABSORBENT ARTICLES AND METHODS OF MANUFACTURING THE SAME." This application also claims priority to PCT Application No. PCTUS1156869 filed October 19, 2011.

### BACKGROUND

The present invention relates generally to absorbent articles and, in particular, to absorbent articles having improved fit, comfort and manufacturability.

Millions of people of all ages suffer from incontinence of the bowel or bladder. Whether an infant, adult, or elderly person, the underlying cause of incontinence varies but the method of treatment typically involves absorbent article products. Adult incontinent briefs, disposable diapers and underpads can alleviate some of the emotional and physical discomfort of incontinence by absorbing and containing liquid and other discharges from the human body to prevent body and clothing soiling.

Despite recent pressure from Center for Medicaid and Medicare Services (CMS) to change clinical practice to promote systematic treatment of incontinence, improper use of absorbent incontinent products for the management of urinary and fecal incontinence continues. The high prevalence of incontinence in nursing home residents results in Incontinence Associated Dermatitis (IAD) in the perineal area and is a common complication. Improper fit or use, applying the wrong size to a user, of absorbent articles is a contributing factor to perineal dermatitis by creating increase heat build-up or chafing against the superficial skin tissue.

In an attempt to improve performance and reduce abrasion to the skin, the absorbent articles industry has been introducing new product concepts with, for example, non-woven back sheets for better comfort. However, these products still contribute to the development of IAD due to the improper size and design in the crotch chaises and leg cut out. In an attempt to size the garments appropriately, a majority of current absorbent articles are sold in five sizes: Medium, Regular, Large, Extra Large and 2X Large. These sizes however do not resolve the above issues as the articles do not sufficiently accommodate users with different body size proportions. As a result caregivers constantly need to use a larger, oversized garment because the product design does not allow for good fit around the patients waist and thigh leg area at the same time. Thus, caregivers are placing larger garments on the patient to compensate which leads to higher cost; potential leakage; and lower dignity because of a bigger bulky garment being worn.

Further, not using the correct size garment on a patient who suffers from incontinence violates the federal governments CMS-F315 rules that require care professionals and staff to use the correct size and type of garment based on the patient's condition and size.

Additionally, multiple sizes are created by multiple size components leading to inefficient manufacturing process. Each size requires the manufacturer to stop the machine and change out a number of the machine's sections in order to produce the next size. After changing the machine sections, other sections need to be recalibrated in order to insure the raw material components are converted correctly. These changeovers can take anywhere between 6 to 12 hours depending on the machine being used by the manufacturer. This downtime reduces the amount of product the machine can produce and increases the manufacturers converting cost. Further, current adult brief machines manufactures are required to use extra steel and other materials in order to build the additional components for the additional sizes.

In another attempt to improve performance and fit, incontinence articles are configured with stretchable material attached to the back panel of the article's ears. However the stretch briefs have shorter front panels in order to offset the cost of stretch material. First this requires the caregiver to use a different application technique because the attachment zone is smaller. Second, the stretch material, when extended fully to accommodate a larger waist for patients who at the upper limit of sizing, can cause skin irritation and breakdown, particularly in the waist and hip region. Often the waist securement portions are over-stretched and incorrectly fit, traumatizing the skin as it lays adjacent to.

In an attempt to improve the performance of an absorbent layer of the incontinence article, the top of a core of the absorbent layer can be embossed. Embossing the top of the absorbent layer can increase the lateral movement of fluid that comes in contact with the absorbent layer and can reduce leakage, but can also increase circular movement of the fluid and increase leakage. Similarly, absorbent layers having two cores have similar advantages and disadvantages. In addition, flow between the top core and the bottom core can be insufficient, even when the top of the bottom core is embossed.

In addition, when donning the product, significant forces may be subjected upon the side panels, especially while the product is being applied and the wearer's body weight is directly on the chassis of the absorbent article. These forces can be localized along one edge of the article, and therefore can cause a failure, for example, a tear in the panel, which propagates quickly and completely across the span of the panel, thereby causing a complete failure of the article.

Additionally, a patient with skin care concerns in the hip region may benefit from a product that can provide softness and a more comfortable side panel. Additionally, as adult brief products have evolved, these products now include side panels that are used for closure. As these side panels are used, it is possible for the wearer to don the product in such a way that the side panel can bunch, and cause pressure lines against the wearer. Additionally, when the product is manufactured, the process of applying the side panel to the diaper includes a manufacturing step of folding a portion of the side panel. If elements of the manufacturing process are not ideal, scrap can be created in the manufacturing process.

Love U.S. Patent No. 9,439,811 (U.S. Patent Application Publication No. 2012/0271267) discloses an absorbent article with a chassis width that changes across size ranges while the chassis length remains constant.

Datta U.S. Patent Application No. 6,315,765 discloses an absorbent pad with elasticized side flaps to impede lateral fluid movement.

Accordingly, a need exists for absorbent articles that provide a better fit and comfort, and increased absorption, while reducing the number of sizes within a range of products, reducing manufacturing costs, and reducing the impact on the environment. A need also exists for absorbent articles that will resist bunching, while at the same time providing softness and the effect of padding for the wearer. A further need exists to improve the ability to fold this side panel during the manufacturing process.

### BRIEF DESCRIPTION OF THE DRAWINGS

The foregoing and other advantages of the invention will become apparent upon reading the following detailed description and upon reference to the drawings.
FIG. 1 illustrates a top view of absorbent article in a first configuration according to one embodiment.
FIG. 2A-B illustrates a top view of a first absorbent article and a second absorbent article in the first configuration according to one embodiment.
FIG. 3A-B illustrates a top view of a third absorbent article and a fourth absorbent article in the first configuration according to one embodiment.
FIG. 4 illustrates a perspective view of an absorbent article in a second configuration.
FIG. 5 illustrates a side view of an absorbent article in a third configuration.
FIG. 6 illustrates a front securement portion coupled to a back securement portion according to one embodiment.
FIG. 7 illustrates a securement portion having an elastic panel according to one embodiment.
FIG. 8 illustrates a wearer including an absorbent article in the third configuration according to one embodiment.
FIG. 9 illustrates a wearer including an absorbent article in the third configuration according to another embodiment.
FIG. 10 illustrates an exploded view of the absorbent article shown in FIG. 1.
FIG. 11 illustrates a perspective view of a second core of the absorbent article depicted in FIG. 1.
FIG. 12 illustrates a perspective view of a first core of the absorbent article depicted in FIG. 1.
FIG. 13 illustrates a cross-section of an end view of a portion of an absorbent article according to an embodiment.
FIG. 14 illustrates a cross-section of an end view of a portion of an absorbent article according to an embodiment.
FIG. 15 illustrates a cross-section of an end view of a portion of an absorbent article according to an embodiment.
FIG. 16 illustrates a cross-section of a side view of a portion of the absorbent article shown in FIG. 15.
FIG. 17 illustrates a securement portion having a lamination layer according to one embodiment.
FIG. 18 illustrates a securement portion having a lamination layer according to a further embodiment.
FIG. 19 illustrates a securement portion having a lamination layer according to a further embodiment.
FIG. 20 illustrates a cross section of securement portion having a lamination layer according to the embodiment of FIG. 19.
FIG. 21 illustrates a securement portion having a lamination layer according to a further embodiment.
FIG. 22 illustrates a cross section of securement portion having a lamination layer according to the embodiment of FIG. 21.
FIG. 23 illustrates a securement portion having a lamination layer according to a further embodiment.
FIG. 24 illustrates a cross section of a folded absorbent article having a securement portion according to the embodiment of FIG. 23.
FIG. 25 illustrates an embodiment of a bonding pattern for a securement portion having a lamination layer.
FIG. 26 illustrates a top view of an embodiment of an absorbent article including a sacral lamination layer.
FIG. 27 illustrates a schematic of a portion an embodiment of a method for manufacturing an absorbent article with a rear side panel in accordance with embodiments shown in FIGS. 17-25.
FIG. 28 illustrates a schematic of a further portion an embodiment of a method for manufacturing an absorbent article with a rear side panel in accordance with embodiments shown in FIGS. 17-25.
FIG. 29 illustrates a schematic of a further portion an embodiment of a method for manufacturing an absorbent article with a rear side panel in accordance with embodiments shown in FIGS. 17-25.

While the invention is susceptible to various modifications and alternative forms, specific embodiments have been shown by way of example in the drawings and will be described in detail herein.

### DESCRIPTION OF ILLUSTRATIVE EMBODIMENTS

Absorbent articles as described herein generally include a moisture-pervious inner layer, an absorbent layer, and a moisture-impervious outer layer. Although the remainder of the description will be specifically directed to adult incontinence articles, a disposable diaper, it is to be understood that the embodiments may also be implemented on other absorbent articles, baby diapers for example, and that the properties and uses described below apply to these other absorbent articles as well.

Embodiments of the invention are now described in detail. Referring to the drawings, like numbers indicate like parts throughout the views. As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, and the like may be used solely to distinguish one entity or action from another entity or action without necessarily requiring or implying any actual such relationship or order between such entities or actions. Also, reference designators shown herein in parenthesis indicate components shown in a figure other than the one in discussion. For example, talking about a device (10) while discussing figure A would refer to an element, 10, shown in figure other than figure A.

In some embodiments, an absorbent article includes a chassis. The chassis has a substantially rectangular shape including a length extending in a longitudinal direction from the back to the front of a user and a width extending in a lateral direction substantially perpendicular to the length between first and second longitudinal edges. The chassis further includes a first portion, a second portion and a crotch portion extending between the first and second portion, a portion of the chassis being configured to absorb fluids. The absorbent article includes a first securement portion operatively coupled to the first portion of the chassis, the first securement portion having a width extending in a lateral direction from the first longitudinal edge of the chassis first portion and a length extending longitudinally along the first longitudinal edge. The absorbent article includes a second securement portion operatively coupled to the second portion of the chassis in a position longitudinally spaced from the first securement portion and configured to releasably attach to the first securement portion. The chassis is configured to include one of a first chassis length or a second chassis length. When the chassis includes the first length, the chassis includes a first width and the first securement portion includes a second width, and when the chassis includes the second length, the chassis includes the first width and the first securement portion includes a third width, greater than the second width..

In some embodiments, an absorbent article includes a chassis. The chassis including a length extending in a longitudinal direction from the back to the front of a user and a width extending in a lateral direction substantially perpendicular to the length between first and second longitudinal edges. The chassis comprises a first portion, a second portion and a crotch portion extending between the first and second portion, a portion of the chassis being configured to absorb fluids. The absorbent article includes a first securement portion operatively coupled to the first portion of the chassis, the first securement portion having a width extending in a lateral direction from the first longitudinal edge of the first portion of the chassis and a length extending longitudinally along the first longitudinal edge. The absorbent article includes a second securement portion having a stretch portion and a non-stretch portion, the second securement portion operatively coupled to the second portion of the chassis in a position longitudinally spaced from the first securement portion and configured to releasably attach to the first securement portion. The chassis configured to include one of a first length or a second length. When the chassis includes the first length, the stretch portion of the second securement portion includes a first width and the non-stretch portion of the second securement portion includes a second width, and when the chassis includes the second length, the stretch portion of the second securement portion includes the first width and the non-stretch portion of the second securement portion includes a third width, greater than the second width..

In some embodiments, a system of absorbent articles to ensure a properly fitting absorbent article includes a first absorbent article and a second absorbent article. The first absorbent article has a first width and a first length along a longitudinal edge. The first absorbent article includes a first portion, a second portion, and a crotch portion extending between the first and second portion. The first absorbent article includes a securement portion operatively coupled to the first portion, the securement portion having a width extending in a lateral direction from the longitudinal edge of the first portion and a length extending longitudinally along the longitudinal edge. The second a second absorbent article has the first width and a second length along a longitudinal edge. The second absorbent article includes a first portion, a second portion, and a crotch portion extending between the first and second portion. The second absorbent article includes a securement portion operatively coupled to the first portion, the securement portion having a width extending in a lateral direction from the first longitudinal edge of the first portion and a length extending longitudinally along the first longitudinal edge.

Other features further provide leg openings that are more generous as a result of the rectangular chassis, rectangular body securement portion configuration allowing for a more accurate fit to accommodate a wide range of body types per article size. Front panels are configured to underlay the back panels providing a more comfortable fit and reduced skin degradation and trauma to the wearer.

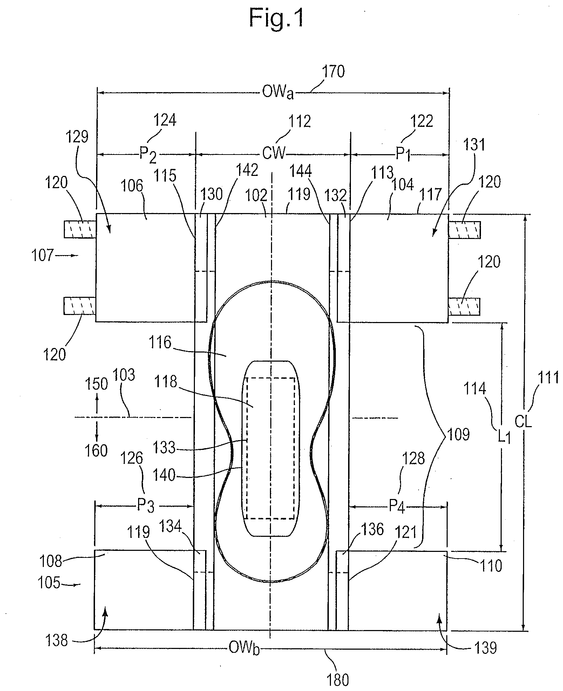

FIG. 1 illustrates in plan view, an exemplary non-limiting general embodiment of an absorbent article 100 in a substantially flat un-contracted state (the first configuration), having a reduced component configuration that accommodates multiple sized wearers. In this embodiment the article comprises a chassis 102, securement portions 104, 106, 108, 110, a first core portion 116, and a second core portion 118. Although not shown in this figure, the absorbent article 100 may include a set of leak guards and/or a set leg cuffs 142, 144, both known to those of ordinary skill in the art. In this embodiment there are four body securement portions comprising a first securement portion 104, a second securement portion 106, a third securement portion 108, and a fourth securement portion 110. It should be noted that four securement portions are used in this embodiment however it should be recognized that other embodiments may be configured with more or fewer securement portions.

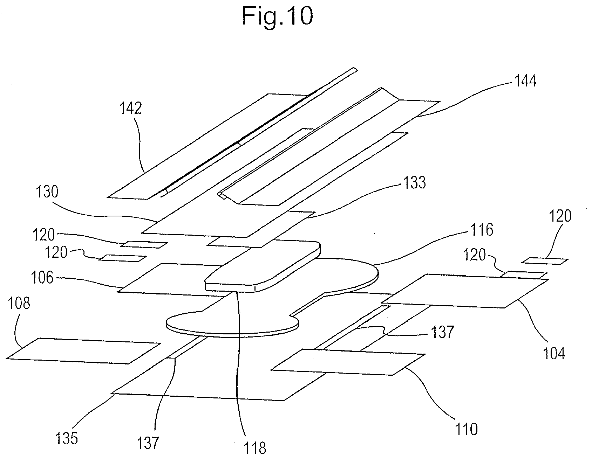

The absorbent article 100 generally consists of several layers (see, e.g., FIG. 10), including an inner layer, an absorbent layer, and an outer layer. The inner layer faces a wearer and contacts the skin of the wearer when the absorbent article 100 is secured to the wearer. The inner layer may be composed of a moisture-pervious fabric suitable to allow bodily discharge to pass through the inner layer and be absorbed by the absorbent layer. Non-limiting examples of materials suitable to form the inner layer include polypropylene, polyethylene, polyester, materials having hydrophobic properties, combinations thereof and/or the like. Additionally, the inner layer can be treated with a hydrophilic finish to improve pass through of liquids to diaper layers beneath the inner layer. Non-limiting examples of suitable hydrophilic finishes include stearic acid, melamine-based chemicals, fluorocarbon chemicals, and silicon based chemicals.

The absorbent article 100 generally has a back region 150 and a front region 160. First securement portion 104 and second securement portion 106 are coupled to and may extend from the back region 150, and third securement portion 108 and fourth securement portion 110 are coupled to and may extend from the front region 160. The back region 150 is generally positioned against the back of the user. The front region 160 is generally positioned against the front of the user. The third securement portion 108 and the fourth securement portion 110 are configured to wrap around a wearer's waist from front to back, and the first securement portion 104 and the second securement portion 106 are configured to wrap around a wearer's waist from back to front. In this manner, first securement portion 104 and second securement portion 106 can be coupled to third securement portion 108 and fourth securement portion 110, respectively, to couple the front region 160 to the back region 150.

The absorbent article 100 in this illustration of FIG. 1 is shown with the portion of the absorbent article 100 that contacts the wearer shown facing the viewer. The absorbent article 100 includes a longitudinal axis 101 and a lateral axis 103. The absorbent article 100 includes a first end portion 105, a second end portion 107, and an intermediate portion 109. The first end portion 105 is also referred to herein as a front waist region 105, the second end portion 107 is also referred to herein as a back waist region 107, and is substantially opposite the first end portion 105, and the intermediate portion 109 is also referred to herein as a crotch region 109, and is disposed longitudinally between the front and back waist regions 105 and 107. The front waist region 105 and the back waist region 107 generally comprise those portions of the absorbent article 100 which, when worn, encircle the waist of the wearer. The crotch region 109 is that portion of the absorbent article 100 which, when the absorbent article 100 is worn, is generally positioned between the legs of the wearer. The back securement portions 104 and 106 and the front securement portions 108 and 110, are separated by distance L1 114. This distance L1 114 defines a portion of the leg opening.

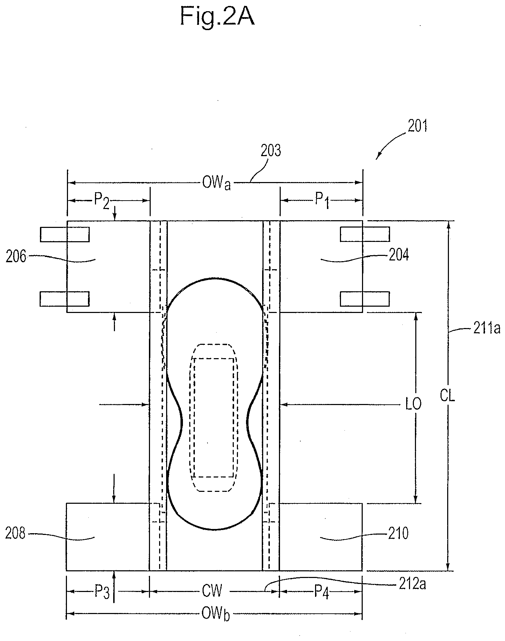

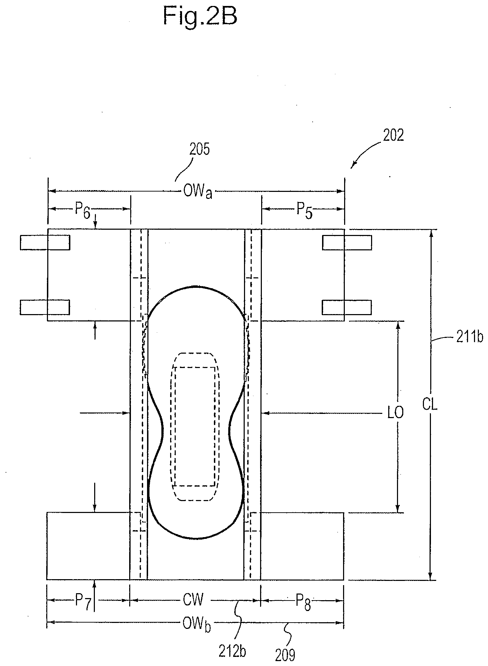

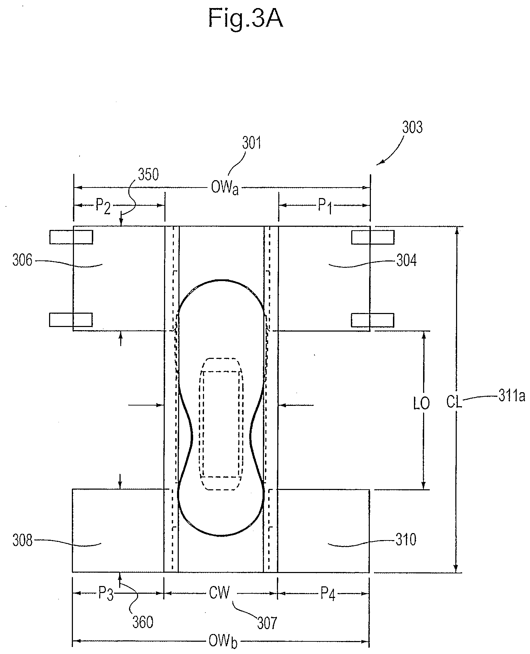

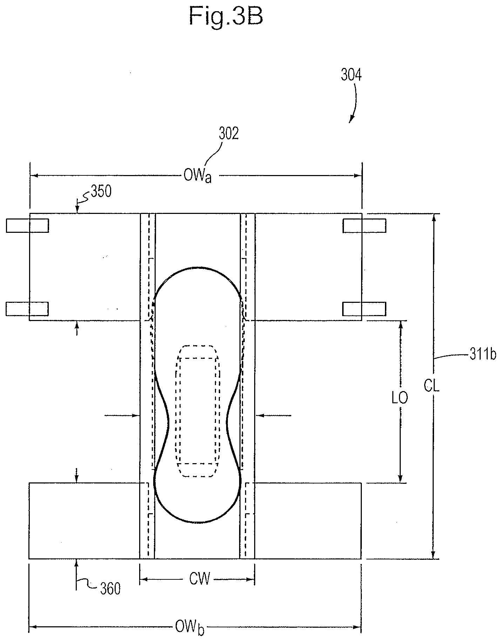

The article's chassis 102 has a chassis width "CW" 112, and a chassis length "CL" 111. The chassis width 112 is a common width across all article sizes that accommodate a plurality of body sizes. Stated in a different way, the absorbent article 100 has a multi-article-size-accommodating width 112 and a multi-article-size-accommodating length 111. Still, put in other words, one chassis width 112 is used for different sized brief articles while still accommodating different wearer's body sizes. By way of example, there are two lengths of the chassis, as illustrated in FIG. 2 and FIG. 3 in combination with FIG.1, a first chassis length CL 211a, 211b for a first article size and a second article size, in this embodiment a Medium size and a Regular size, and a second chassis length CL 311a 311b for a third article size and a fourth article size, for example a Large size and a X-Large size in this embodiment.

The chassis 102 has a shape such that its outer perimeter is rectangular or at least substantially rectangular in the first configuration in this embodiment the absorbent article 100 has at least one securement portion that is coupled to the chassis 102 at one of the front waist region 105 or the back waist region 107. In other embodiment, there may be portions of the chassis that are shaped and/or removed, such as in the crotch region 109, for example, resulting in a narrower crotch region portion 109 to provide a contoured fit between the legs. Still other embodiments have different shaped chassis, such as hour glass shapes, T-shapes, and the like.

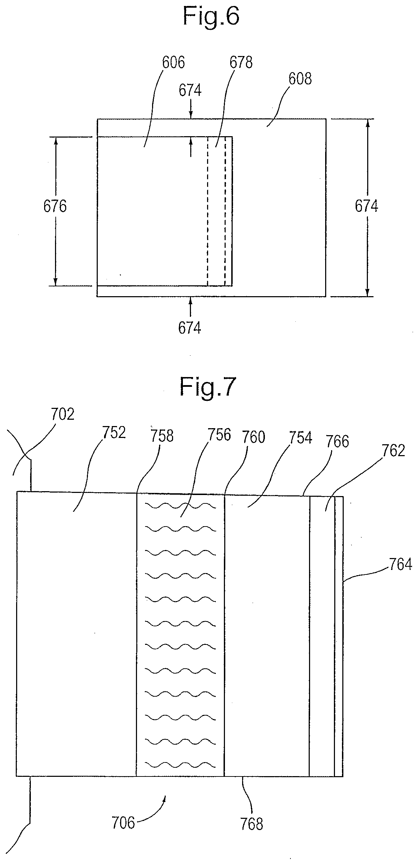

The first securement portion 104 is coupled to a first longitudinal side edge portion 113 of the chassis, the first securement portion 104 overlapping the chassis 102 along the lineal contact points of attachment 132. The amount of overlap is sufficient to reliably attach the securement portion to the chassis such that there is no separation during use, as would be understood to one of ordinary skill in the art. In one embodiment, the overlap of the first securement portion 104 with the chassis 102 longitudinal side edge 113 is between 6mm and 50mm (or about 0.25 inches and 2.0 inches). In this embodiment, the overlap of the securement portion with the chassis is generally the same for all four securement portions. First securement portion 104 includes two fasteners 120. Fasteners 120 can be configured to operatively couple first securement portion 104 to third securement portion 108 and/or to anywhere along the front region 105. While FIG. 1 depicts first securement portion 104 as including two fasteners 120, in some embodiments, first securement portion 104 can include more or fewer fasteners. While FIG. 1 depicts fasteners 120 sized and shaped a particular way, in other embodiments, fasteners 120 can be a different size and/or shape, such as, for example, similar to fastener 762 as depicted in FIG. 7.

The second securement portion 106 is coupled to the second longitudinal side edge portion 115 of the chassis, the first securement portion 106 overlapping the chassis 102 along the lineal contact points of attachment 130, in the same manner as the first securement portion 104. Together, the first securement portion 104, the back waist region of the chassis 107, and the second securement portion 106, laterally form the overall width "OWₐ" 170 of the article at the back region. The first securement portion 104 has a first width P₁ 122 and the second securement portion 106 has a second width P₂ 124. Second securement portion 106 includes two fasteners 120. Fasteners 120 can be configured to operatively couple second securement portion 106 to fourth securement portion 110 and/or to anywhere along the front region 105. While FIG. 1 depicts second securement portion 106 as including two fasteners 120, in some embodiments, second securement portion 106 can include more or fewer fasteners. While FIG. 1 depicts fasteners 120 sized and shaped a particular way, in other embodiments, fasteners 120 can be a different size and/or shape, such as, for example, similar to fastener 762 as depicted in FIG. 7.

In some embodiments, the first securement portion 104 and the second securement portion 106 can be coupled to the chassis 102 in the following manner. An end portion 131 of first securement portion 104 can be temporarily coupled to and end portion 129 of second securement portion 106. In some of these embodiments, end portion 131 can overlap end portion 129 and can be temporarily coupled to end portion 129 via an adhesive. In other of these embodiments, end portion 131 can be temporarily coupled to end portion 129 via a perforated seem (not shown) between end portion 129 and end portion 131. Chassis 102 can be temporarily coupled to first securement portion 104 and second securement portion 106. Specifically, the back waist region 107 of the chassis 102 can be disposed substantially on top of the end portion 129 of the first securement portion 104 and the end portion 131 of the second securement portion 106. Said another way, end portion 129 of first securement portion 104 and end portion 131 of second securement portion 106 can be disposed on a first side of chassis 102. At least a portion of first securement portion 104 can be folded over chassis 102 such that first securement portion 104 can be operatively coupled to chassis 102 approximately at lineal contact points 132 as described above. At least a portion of second securement portion 106 can be folded over chassis 102 such that second securement portion 106 can be operatively coupled to chassis 102 approximately at lineal contact points 130 as described above. End portion 129 and end portion 131 can be released from contact with the other of end portion 129 and end portion 131.

The third securement portion 108 is coupled to a third longitudinal side edge portion 119 of the chassis, the third securement portion 108 overlapping the chassis 102 along the lineal contact points of attachment 134. The amount of overlap is sufficient to reliably attach the securement portion to the chassis such that there is no separation during use, as would be understood to one of ordinary skill in the art.

The fourth securement portion 110 is coupled to the fourth longitudinal side edge portion 121 of the chassis, the first securement portion 110 overlapping the chassis 102 along the lineal contact points of attachment 136, in the same manner as the first, second or third securement portion 104, 106, 108. Together, the third securement portion 108, the front waist region of the chassis 105, and the fourth securement portion 110, laterally form the overall width "OW_{b}" 180 of the absorbent article 100 at the back region. The third securement portion 108 has a third width P₃ 126 and the fourth securement portion 110 has a fourth width P₄ 128. In this embodiment all four securement portion widths P₁ - P₄ are substantially the same.

Third securement portion 108 and fourth securement portion 110 can be operatively coupled to the chassis 102 via temporary coupling of end portion 138 of third securement portion 108 and end portion 139 of fourth securement portion 110 in a manner similar to that described above with reference to first securement portion 104 and second securement portion 106.

In other embodiments the width of the securement portions may vary from portion to portion as well as from front to back or side to side. For example, the first securement portion 104 and the second securement portion 106 may have a substantially equal width but a different width than the third securement portion 108 and the fourth securement portion 110. In another embodiment the first securement portion and the second securement portion are one continuous piece, running across the entire chassis in a perpendicular fashion and extending beyond the edges of the chassis, forming the body securement portions. In another embodiment the third securement portion and the fourth securement portion are one continuous piece, running across the entire chassis in a perpendicular fashion and extending beyond the edges of the chassis, forming the body securement portions. In one embodiment the overall width of the back region "OWₐ" 170, including the first securement portion 104, the chassis 102 and the second securement portion 106 stays the same, and similarly the overall width of the front region "OW_{b}" 180, including the third securement portion 108 the chassis 102 and the fourth securement portion 110 stays substantially the same.

In one embodiment the chassis 102 has a common chassis width CW 112 between 190mm and 350mm and preferably about 320mm for all article sizes (the first size article, the second size article, the third size article and the fourth size article). In this embodiment the width of each securement portion (Pₓ) is the same at the back region end 150 as it is in the front region end 160. For all article sizes (e.g. medium, regular, large extra large), the chassis width 112 is the same. In this embodiment the chassis 102 has a rectangular shape or a substantially rectangular shape, wherein the width is the short dimension. For the first article size and the second article size, the chassis 102 may also have a first common chassis length CL 111 which in this embodiment is between 800mm and 880mm and preferably 860mm. For the third article size and the fourth article size, the chassis 102 may have a second common chassis length CL 111, different from the first common chassis length, and in this embodiment is between 860mm to 1000mm and preferably 960mm. The Chassis length CL 111 in this figure refers to the dimension and not the actual value.

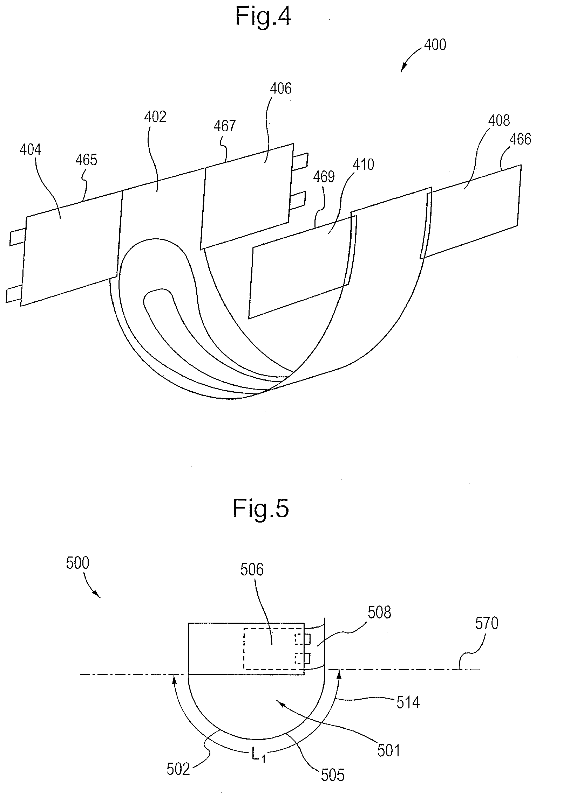

As shown in FIG. 1, the edge 199 of chassis 102 can be substantially flush with edge 117 of portion 104 and portion 106. In some embodiments, the edge 199 can extend beyond the edge 117 of portion 104 and portion 106 (see, e.g., FIG. 4).

The articles are assembled together in the manufacturing process such that the body securement portions are secured to the chassis 102 wherein the panels 104, 106, 108 and 110 are disposed between a first chassis layer, for example a top sheet, and a second chassis layer, for example a bottom sheet. The machine assembling the article is set such that the panel widths are changed from a first width to a second width in order to change over from building a first article size 201 to a second article size 202. Similarly, when changing over to a third article size, the panel widths are increased, and additionally in this embodiment, the chassis length is increased to the second chassis length as for the third article size 303 and fourth article size 304. For these changes, the changeover is completed by running different programs in the machine and minimal or even zero hard tooling change over is required. By way of example, there may be no hard tool changeovers such as the replacement of cutting dies which are typically used for cutting leg openings. Instead, only vacuum plate changes may be needed for the size changes in the panel widths and length which are relatively simple replacements. In some embodiments, the vacuum plates need not even be physically replaced, the vacuum plates are sized to accommodate all sizes of panels and depending on the size of the panel, and vacuum ports are enabled or disable based on the size of the panel.

FIG. 2 and FIG. 3 illustrate together, in plan view fashion, an exemplary non-limiting general embodiment of four different sized absorbent articles, in the flat un-contracted state. The first absorbent article 201 configured as a first wearer size and the second absorbent article 202 configured as a second wearer size are shown in FIG. 2. The third absorbent article 303 sized as a third wearer size and the fourth absorbent article 304 sized as a fourth wearer size are shown, in their flat un-contracted state, in FIG. 3. The different wearer sizes, the first and second articles 201, 202 are created by configuring the securement portion widths, e.g. P1, P2 or both, for the first wearer size 201 and the second wearer size 202 in order to accommodate different wearer waist sizes, all with the same chassis width 212 and chassis length 211a and 211b. For example, the first size article 201 will have a fixed chassis width "CW" of about 320mm and first, second, third and fourth securement portion individual widths of about 180mm, while the second size article will have first, second, third and fourth securement portion widths of about 200mm, however still with the chassis width "CW" of 320mm. The chassis length 211a and 211b, are about 860mm for the first and second articles 201, 202.

In this embodiment, using the first size article 201 as an example, the securement portion width "Pₓ" may be maintained the same for each securement portion 204, 206, 208, 210 or in another embodiment the first securement width 204 may be a different width than the second securement portion width 206, while maintaining the same overall width 203 of the rear portion of the article. In yet another embodiment the first and second securement portions 204, 206, may have a first width, and the third and fourth securement portions 208, 210 may have a second width.

For the third size 303 and the fourth size 304 (FIG. 3), the chassis width 311a and 311b "CW" of about 320mm, remains the same as the first and second sizes, however the securement portion widths "Pₓ" and the chassis lengths "CL" 111 are configured differently from the first size 201 and the second size 202 to accommodate the different, greater in this embodiment, article sizes. The third size article 303 will have a fixed chassis width "CW" of about 320mm and first, second, third and fourth securement portion widths of about 255mm, while the fourth size article 304 will have first, second, third and fourth securement portion widths of about 275mm. The chassis length, 311a and 311b may be between 860mm and 1000mm and in one embodiment the chassis length 311a and 311b, is about 960mm for the first and second articles 201, 202.

As with the first and second article sizes 201, 202, the securement portion width "Pₓ" may be maintained the same for each securement portion 304, 306, 308, 310 or in another embodiment the first securement portion 304 width may be a different width than the second securement portion width 306, yet while maintaining the same overall width "OWₐ" 301 of the article at the back end or the same overall width "OW_{b}" 302 of the article at the front end. In yet another embodiment the first and second securement portions 304, 306, may have a first width, and the third and fourth securement portions 308, 310 may have a second width. As the width of the first and second portions increases, the width of the third and fourth portion may decrease, so long as there is overlap in accordance with the size of the article to fit the wearer.

In the embodiment shown in FIG. 3, the third size article 303 will have a fixed chassis width "CW" of about 320mm and first, second, third and fourth securement portion widths of about 255mm, while the fourth size article 304 will have first, second, third and fourth securement portion widths of about 275mm. The chassis length, 311a and 311b may be between 860mm and 1000mm and in one embodiment the chassis length 311a and 311b, is about 960mm for the first and second articles 201, 202.

FIG. 4 is a perspective view of an absorbent article 400 in a second configuration. Absorbent article 400 can be similar to and include similar components as absorbent article 100. By way of example, absorbent article 400 includes securement portion 404, 406, 408, and 410, which can be similar to securement portions 104, 106, 108, and 110, respectively. The second configuration shows the chassis 402 in a position as it would be when placed on the wearer although the securement portions, 404, 406, 408 and 410 remain in an un-contracted, unwrapped state. Securement portion 404 includes an edge 465, securement portion 406 includes an edge 467, securement portion 408 includes an edge 466, and securement portion 410 includes an edge 469.

FIG. 5 is a side view of an absorbent article 500 in a third configuration. Absorbent article 500 can be similar to and include similar components as absorbent article 100. By way of example, absorbent article 500 includes securement portion 504 (not shown), 506, 508, and 510 (not shown), which can be similar to securement portions 104, 106, 108, and 110, respectively. The third configuration shows the securement portions in a wrapped state, as they would be wrapped around a wearer. In this embodiment, the securement portions may be configured such that the second securement portion 506 overlaps the third securement portion 508, and the first securement portion 504 overlaps the fourth securement portion 510. The third and fourth securement portions 508, 510, lay directly adjacent the wearer's skin, while the first securement portion 504 and the second securement portion 506, lie partially on the wearer's skin and partially on the third and fourth securement portions respectively. Having front securement portions that may wrap substantially up to an in some embodiments substantially past a person mid point and around towards and against the wearer's backside provides a more comfortable fit.

The leg opening 501, is defined by a bottom edge 570 of the securement portions, a second securement portion 506 and a third securement portion 508, and the longitudinal edges portions 505 of the chassis 502, having the length L1 114 between the securement portions along the longitudinal edge of the chassis 502. Because of the rectangular nature of the securement portions 506 and 508 for the first leg opening, the bottom edge 570 is significantly adjacent the waist and, is positioned higher than or at least significantly above the thigh of the wearer.

FIG. 6 illustrates a front securement portion 608 and a back securement portion 606. The front securement portion 608 can be similar to third securement portion 108 and fourth securement portion 110, and back securement portion 606 can be similar to first securement portion 104 and second securement portion 106. The front securement portion has a length 674 and the back securement portion has a length 676. The length of the front securement portion 608 is greater than the length 676 of the back securement portion. A fastener 678 is coupled to the back securement portion 606. The length of the front securement portion 608 is greater than the length for the back securement portion 606 to move up or down during fastening and still provide a landing zone for the fastener 678, such that the fastener 678 does not come in contact with the wearer's skin. In this embodiment, the fastener 678 has substantially the same length as the back securement portion 606 length 676. The fastener 678 in this embodiment may be applied to the back securement portion 606 and cut to length during the formation of the back securement portion 606.

FIG. 7 illustrates a securement portion 706, of a chassis 702, the securement portion 706 including an elastic panel coupled thereto. Securement portion 706 can be similar to and include similar components with first securement portion 104 and second securement portion 106. In one embodiment, the securement portion 706 has a first non-elastic panel 752, a second non-elastic panel 754 and an elastic panel 756. In one embodiment, the elastic panel 756 is disposed between the first non-elastic panel 752 and the second non-elastic panel 754. Each panel has a first side edge and a second side edge, distal the first side edge, and a top edge 766 and a bottom edge 768, forming a rectangle. The first non-elastic panel 752 is attached to the chassis 702 at a first side edge of the first non-elastic panel 752 and to a first side edge of the elastic panel 756 at a second side edge, distal the first side edge. A second side edge of the elastic panel 756, distal the first side edge, is coupled to a first side edge of the second non-elastic panel 754. The second side edge 764, distal the first side edge, has at least one fastener 762 coupled thereto. Securement portion 706 is coupled to the chassis 702 in a similar fashion to the securement portions 104, 106, 108, 110 as discussed above.

A first seam 758 is formed by the first non-elastic panel 752 second side edge joint with the first side edge of the elastic panel 756. A second seam 760 is formed by the second side edge of the elastic panel 756, joint with the first side edge of the second non-elastic panel 754.

Having a portion of the securement potion 706 include a stretchable material allows for a single article to fit a greater number size rage of wearers. For example in one embodiment, only to article size are need to fit the same size range of wears for the four articles discussed above. This further reduces waste as there is a reduction in the amount of machinery need to build multiple size accommodating articles as well as less change over from product size to product size, thereby increasing efficiency in the manufacturing process.

In one embodiment the elastic panel 756 has a width between 40mm and 100mm and preferably has a width of about 68mm in one embodiment for a first article size. The non-elastic portions may have equal widths which may be about 77mm for example for the first article size. For a second size in this embodiment, the elastic panel 756 has a width between 40mm and 100mm and preferably has a width of about 68mm in one embodiment for a first article size. The non-elastic portions may have equal widths which may be about 108mm for example. There is an overlap of the non-elastic portion and the elastic portion of about 15mm in this embodiment. This overlap is where the two portions are joined together. In this embodiment, the two portions are glued together. One of ordinary skill in the art will understand the plurality of methods and procedures for affixing the portions together.

The stretch material may be made from stretch material as understood by those of ordinary skill in the art. In one embodiment the stretch material is a 107 GSM stretch material by 3M.

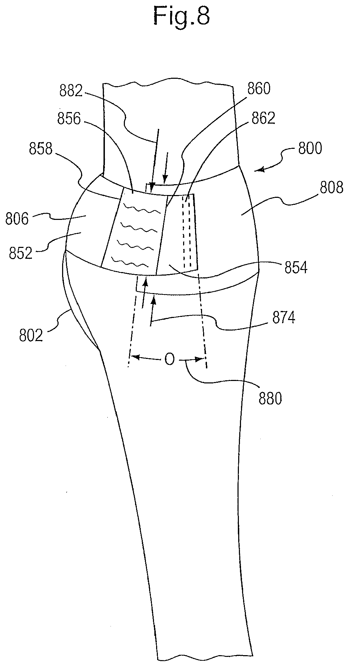

FIG. 8 illustrates a further embodiment of an absorbent article 800 as used by a wearer. The absorbent article can include a chassis 802. In this embodiment a front securement portion 808 wraps around the wearer from front to back and lays underneath the back securement portion 806. The back securement portion 806 can overlap the front securement portion 808 a distance 880. Front securement portion 808 can be similar to and include similar components with third securement portion 108, fourth securement portion 110, and front securement portion 708. Similarly, back securement portion 806 can be similar to and include similar components with first securement portion 104, second securement portion 106 and back securement portion 706. In this embodiment the front securement portion 808 length 874 is greater than the back securement portion 806 length 882. The back securement portion 806 includes an elastic panel 856 disposed between a first non-elastic panel 852 and a second non-elastic panel 854, and includes a fastener 862 similar to fastener 762 and fastener 678. A first seam 858 is formed by the joint between the elastic portion 856 and the first non-elastic portion 852. A second seam 868 is formed by the joint between the elastic portion 856 and the second non-elastic portion 854. The width of the front securement portion 808 is great enough that it lies between the second seam 860 and the wearer's skin reducing the abrasive effect of the seam on the skin. In this embodiment the front securement portion width is between 130mm and 260mm and is preferably 180mm for a first embodiment first size. The overall width for the first size is 680mm, and may be between 630mm and 830mm. The front securement portion width is between 205mm and 335mm and is preferably 255mm for a first embodiment second size. The overall width for the second size is 829mm, and may be between 720mm and 950mm.

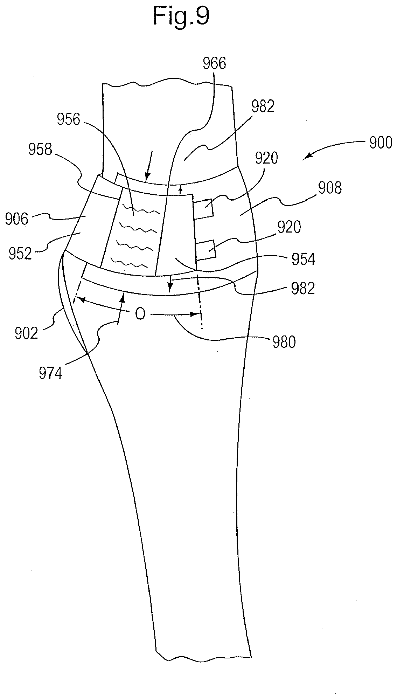

FIG. 9 illustrates a wearer including an absorbent article 900 in the third configuration. In this embodiment a front securement portion 908 wraps around the wearer from front to back and lays underneath the back securement portion 906. The back securement portion 906 can overlap the front securement portion 908 a distance 980. Front securement portion 908 can be similar to and include similar components with third securement portion 108, fourth securement portion 110, and front securement portion 708. Similarly, back securement portion 906 can be similar to and include similar components with first securement portion 104, second securement portion 106 and back securement portion 706. In this embodiment the front securement portion 908 length 974 is greater than the back securement portion 906 length 982. The back securement portion 906 includes an elastic panel 956 disposed between a first non-elastic panel 952 and a second non-elastic panel 954, and includes a fastener 920 similar to fastener 120. A first seam 966 is formed by the joint between the elastic panel 956 and the second non-elastic panel 954. The width of the front securement portion 908 is great enough that it lies between the first seam 966 and the wearer's skin reducing the abrasive effect of the seam on the skin. A second seam 958 is formed by the joint between the elastic panel 856 and the first non-elastic panel 852. The width of the front securement portion 908 is great enough that it lies between the first seam 966 and the second seam 958 and the wearer's skin reducing the abrasive effect of the seam on the skin.

In this embodiment the front securement portion width is between 130mm and 260mm and is preferably 180mm for a first embodiment first size. The overall width for the first size is 680mm, and may be between 630mm and 830mm. The front securement portion width is between 205mm and 335mm and is preferably 255mm for a first embodiment second size. The overall width for the second size is 829mm, and may be between 720mm and 950mm.

FIG. 10 is an exploded perspective view of the absorbent article 100 with certain items removed for clarity. As shown in FIG. 10, absorbent article 100 includes securement portions 104, 106, 108, 110; first core 116; second core 118; fasteners 120; and leg cuffs 142, 144. Additionally, absorbent article includes an inner layer 130, an AD 133 disposed between the inner layer 130 and the second core 118, an outer layer 135, and elastic bands 137. The elastic bands 137 can improve the fit of the absorbent article 100 and can improve the comfort of the wearer.

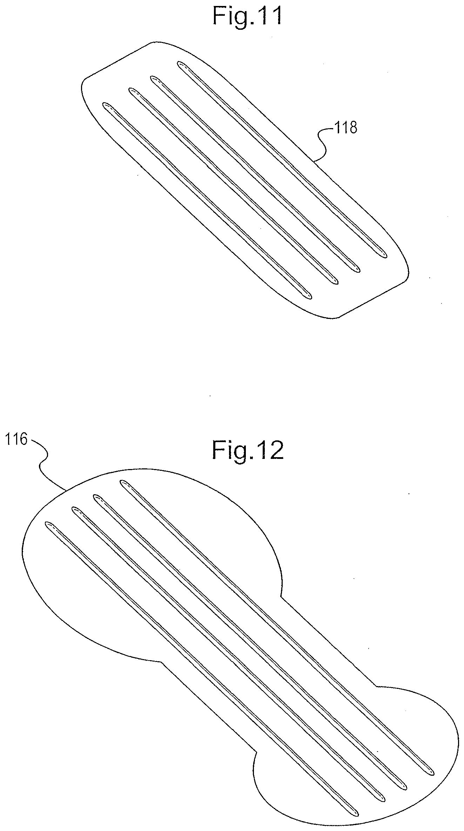

FIG. 11 is a perspective view of a top side (facing towards wearer) of the second core 118, and FIG. 12 is a perspective view of a bottom side (facing away from a wearer) of the first core 116. Each of first core 116 and second core 118 can be composed of similar material, and can be shaped depending on the size of the absorbent article, and whether it is indented for use by infants, children and/or adults. By way of example, and as shown in FIGS. 11 and 12, first core 116 can be larger and substantially hourglass shaped, whereas second core 118 can be smaller, relative to first core 116, and can be substantially rectangular shaped. In this manner, the absorbent article can include a large surface area of coverage provided by the first core 116, and the increased absorbency provided by the second core 118, without the additional bulk of a second core having the same size as the first core.

First core 116 is shown having an embossed bottom and second core 118 is shown having an embossed top. The embossed top of second core 118 and the embossed bottom of first core 116 provide increased longitudinal flow while reducing lateral flow, and, in this manner, reducing leakage. Said another way, the embossed top of second core 118 and the embossed bottom of first core 116 allows fluid to move longitudinally towards the front and the back of a wearer, as opposed to towards the legs of a wearer.

Each of the first core 116 and the second core 118 may be composed of any materials suitable for absorbing the fluids and discharge including, but not limited to, a fibrous material (e.g., fluffed wood pulp), a super absorbent polymer (SAP), or the combination of SAP and fibrous material. The SAP can be natural or synthetic and may be biodegradable. Non-limiting examples of SAP include polymers based on acrylate(s) such as sodium acrylate, potassium acrylate, and/or an alkyl acrylate(s) (e.g., methyl acrylate, ethyl acrylate, propyl acrylate, butyl acrylate, and hexyl acrylate). The absorbency of the diaper may vary depending upon whether it is intended for use by infants, children and/or adults.

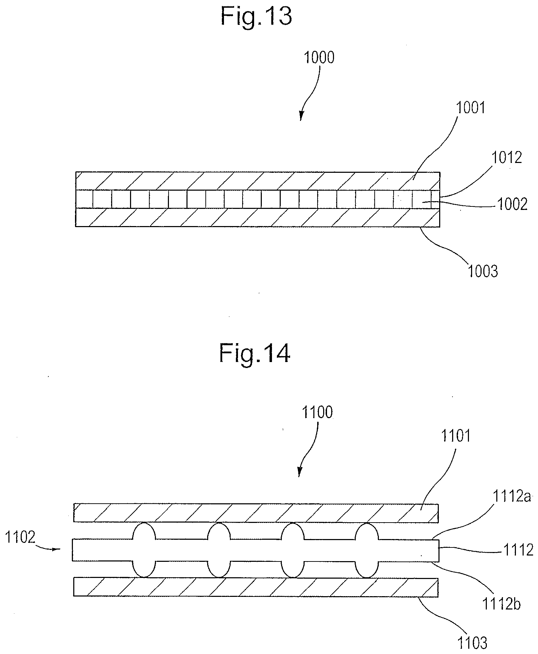

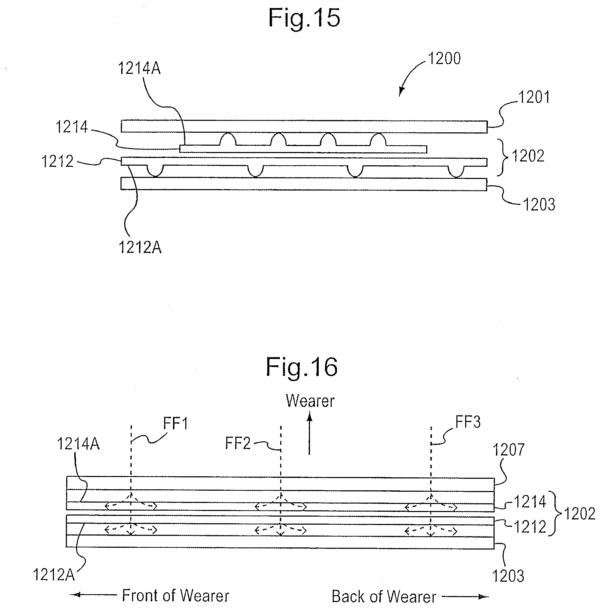

While FIGS. 11 and 12 depict the first core 116 having an embossed bottom, and the second core 118 having an embossed top (see also, for example, FIGS. 15 and 16), in some embodiments, an absorbent article can have only a single core with no embossing (see, e.g., FIG. 13), a single core with embossing on both sides (see, e.g., FIG. 14), and/or other combinations of one or two cores each with embossing on one, both, or neither side. While the FIGS. show absorbent articles include one or two cores, in some embodiments, absorbent articles can include more or fewer cores. While FIGS. 11, 12, 14, and 15 depict embossing as including four spaced apart embossing "lines," in some embodiments, a core can include more or fewer embossing lines. In some embodiments, embossing lines can be adjacent one another, or can be a combination of adjacent and space apart embossing line. In this manner, the different combinations of embossing lines can define an embossing pattern. While FIGS. 11, 12, 14, and 15 depict embossing substantially along the entire width and length of each respective core, in some embodiments a core can have embossing substantially along an entire width and/or length, and/or a portion of a width and/or length.

FIG. 13 is a cross-sectional view of a portion of an absorbent article 1000. The absorbent articles described above generally consists of several layers including an inner layer 1001, an absorbent layer 1002, and an outer layer 1003, as depicted in FIG. 10. In general and applicable to any of the above embodiment, an absorbent layer 1002 may be positioned between the inner layer 1001 and the outer layer 1003 of the absorbent article. The absorbent layer 1002 includes a core 1012. The core 1012 can be similar to and include similar characteristics with one or both of first core 116 and/or second core 118.

The outer layer 1003, which faces away from the wearer when the absorbent article is secured to the wearer, is composed of a moisture-impervious fabric. Accordingly, the outer layer 1003 may be made of any material suitable to minimize or prevent fluids and other discharge from escaping the diaper. Non-limiting examples of suitable materials for the outer layer include polyethylene and/or breathable poly. According to some embodiments, the outer layer can be a thin film such as, for example, polyethylene film. As will be discussed in greater detail below, the outer layer is typically formed from a plastic resin of any of the above-referenced materials. This outer layer 1003 that prevents diapers from leaking also prevents air circulation, thus creating a warm, moist environment where bacteria and fungi can thrive. This bacteria and fungi can cause infectious diseases, unpleasant odors, urinary tract infections, bladder infections, kidney infections, diaper rashes and the like.

FIG. 14 is a cross-sectional view of a portion of an absorbent article 1100. The absorbent article 1100 can be similar to and include similar components with the absorbent article 1000. The absorbent article 1100 includes an inner layer 1101, an absorbent layer 1102, and an outer layer 1103. In general and applicable to any of the above embodiment, an absorbent layer 1102 may be positioned between the inner layer 1101 and the outer layer 1103 of the absorbent article. The absorbent layer 1102 includes a core 1112. Unlike the absorbent article 1000, the core 1112 includes and embossed top 1112A and an embossed bottom 1112B. In this manner, the core 1112 provides increased longitudinal movement of fluid on both the embossed top 1112A and embossed bottom 1112B of the core 1012, and reduced lateral flow, decreasing the potential for leaks from the side of the absorbent article 1100.

FIG. 12 is a cross-sectional end view of a portion of an absorbent article 1200. The absorbent article 1200 can be similar to and include similar components with the absorbent article 1000 and absorbent article 1100. The absorbent article 1200 includes an inner layer 1201, an absorbent layer 1202, and an outer layer 1203. In general and applicable to any of the above embodiment, the absorbent layer 1202 may be positioned between the inner layer 1201 and the outer layer 1203 of the absorbent article. The absorbent layer 1202 includes a first core 1212 and a second core 1214. Unlike the absorbent article 1000, but similar to absorbent article 100, the first core 1212 includes an embossed bottom 1212A and the second core 1214 includes an embossed top 1214A. In this manner, the first core 1212 and the second core 1212B provide increased longitudinal movement of fluid on both the embossed top 1214A and embossed bottom 1212B of the second core 1112 and the first core 1214, respectively.

FIG. 16 is a side view of absorbent article 1200. As depicted in FIG. 16 fluid may flow from a wearer into contact with absorbent article 1200. Fluid flow is shown in FIG. 16 as dashed paths FF1, FF2, and FF3. While fluid flow is shown as dashed paths in FIG. 16, in some embodiments, fluid may not flow along a defined path, or paths, in any pattern, and may or may not contact absorbent article at any one or multiple particular locations. The fluid can first flow into contact with, be partially absorbed by, and pass through, inner layer 1201. The fluid can flow into contact with the embossed top 1214A of second core 1214. A portion of the fluid can be absorbed by second core 1214 and/or move longitudinally and/or laterally on and/or within second core 1214; and a portion of the fluid can pass through second core 1214 and into contact with first core 1212. Both the greater surface area of the embossed top 1214 A, and the space created by embossed top 1214A between inner layer 1201 can direct the fluid flow longitudinally towards the front and the back of the wearer, and away from edges of absorbent article 1200 (e.g., reduced lateral flow. In this manner, when longitudinal flow is increased and/or when lateral flow is decreased, leakage can be reduced. A portion of the fluid can be absorbed by first core 1212 and/or move longitudinally and/or laterally on and/or within first core 1212; and a portion of the fluid can pass through first core 1212 and into contact with outer layer 1203. Both the greater surface area of the embossed bottom 1212 A, and the space created by embossed bottom 1212A between outer layer 1203 can direct the fluid flow longitudinally towards the front and the back of the wearer, and away from edges of absorbent article 1200 (e.g., reduced lateral flow. In this manner, when longitudinal flow is increased and/or when lateral flow is decreased, leakage can be reduced.

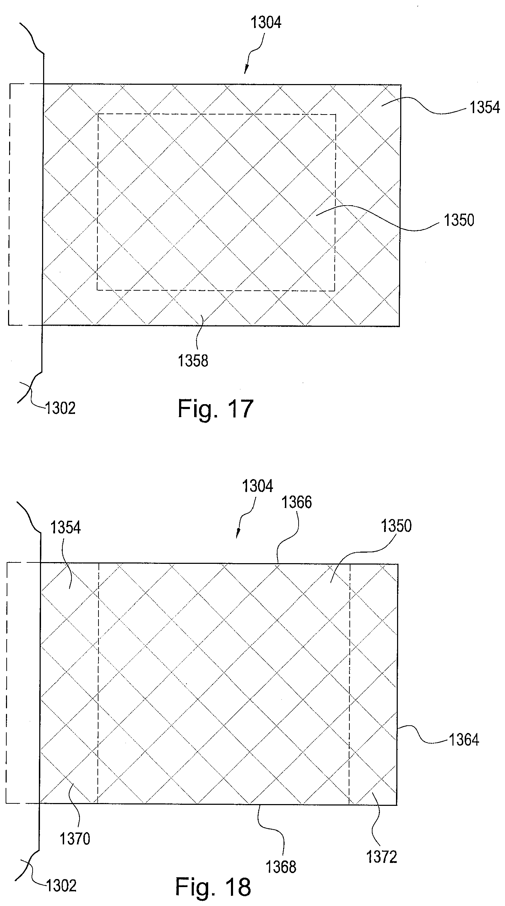

FIGS. 17-25 illustrate a securement portion, also referred to as a side panel, 1304 for connection with a chassis 1302, the securement portion 1304 including a lamination layer 1350 incorporated therein. Securement portion 1304 can be similar to and include similar components with side panel securement portions 104, 106, 108, 110. Accordingly, features of the side panel 1304 may be incorporated into either a back securement portion or a front securement portion or both. However, it will be readily apparent to one of ordinary skill in the art that a side panel consistent with the examples shown in FIGS. 17-25 is not limited to the geometry of shown in these illustrative embodiments. Rather, one of ordinary skill will recognize that any of a broad variety of different side panel shapes, sizes and geometries may be appropriate.

Lamination layer 1350 may comprise a variety of different materials, including a combination of materials, that may provide various benefits as would be recognized by one of ordinary skill in the art. For example, lamination layer 1350 may comprise a high-loft nonwoven material to provide a softer side panel or an absorbent material to provide additional protection against leakage. Alternatively, the lamination layer may include one or more of the following materials: foam, airlaid, tissue, pulp, SAP, coform and meltblown material. In one embodiment, the lamination layer comprises a polypropylene or polyester nonwoven with a basis weight between 10 and 75 GSM, and more preferably 48 GSM.

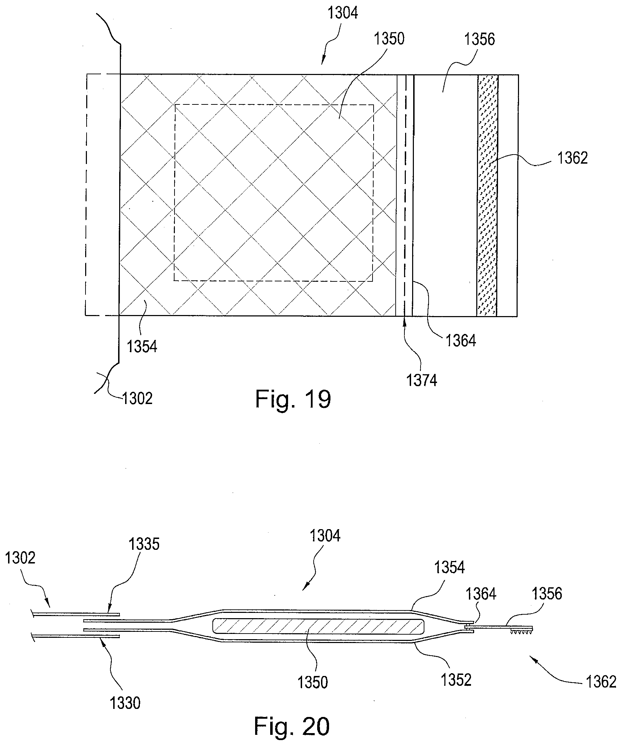

In one embodiment, as illustrated by FIGS. 17 and 20, the side panel 1304 comprises a first patient-facing layer 1354 and a second outwardly facing layer 1352. A lamination layer 1350 may be positioned between the first 1354 facing layer and second 1352 facing layer. In a preferred embodiment, the lamination layer has a higher loft than either the patient-facing layer or outwardly facing layer. In addition, inclusion of the lamination layer 1350 in the side panel 1304 and the resulting laminate structure allows the use of materials for the facing layers 1352, 1354 that may not otherwise be appropriate for use without the lamination layer, such as softer or more flexible materials. In one embodiment, the facing layers comprise, for example, 10 to 20 GSM SMS nonwoven materials. Other appropriate facing layer materials may include spunbond or spunlaced nonwoven materials from polypropylene or polyethylene.

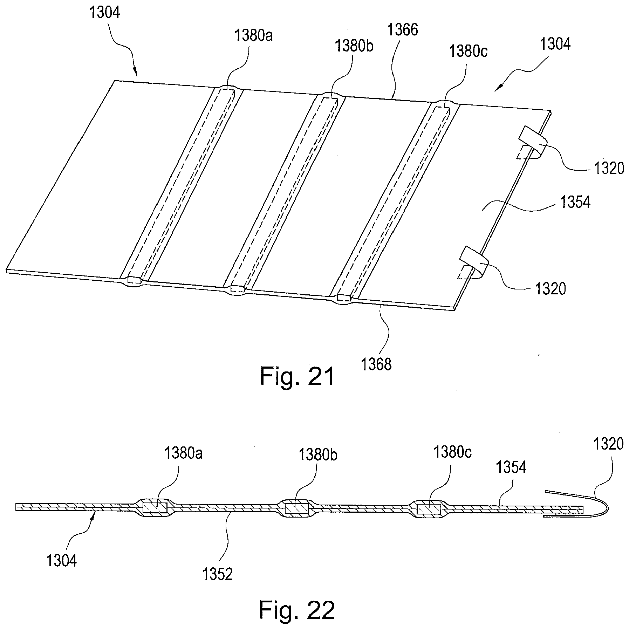

In the embodiment shown in FIG. 17, the lamination layer 1350 has a perimeter that is smaller than the perimeter of the first and second layers such that a portion 1358 of the side panel 1304 without a lamination layer completely surrounds the lamination layer. Alternatively, in the embodiment shown in FIG. 18, the lamination layer 1350 extents from a side panel top edge 1366 to a side panel bottom edge 1368 such that a first portion 1370 without a lamination layer is formed adjacent the chassis 1302 and a second portion 1372 without a lamination layer is formed adjacent the distal edge 1364 of the side panel. In a further embodiment, shown in FIGS. 21-22, alternating strips of lamination layer 1380a, 1380b 1380c are positioned between panel layers 1352, 1354. These strips 1380 may extend from panel top edge 1366 to panel bottom edge 1368. Alternatively, the strips may extend only part of the distance from top edge to bottom edge. If will be understood by one of ordinary skill in the art that, consistent with the present invention, the lamination layer may be arranged in any of a broad variety of continuous or intermittent layouts of which the embodiments discussed herein are only examples.

FIGS. 19-20 illustrate a further embodiment in which the side panel 1304 further incorporates an extension portion 1356 extending beyond the first and second layers 1352, 1354. This extension portion 1356 may comprise a stretchable material, such as an elastic panel. The extension may be glued or bonded 1374 along the distal edge 1364 of the side panel 1304. The extension may further comprise at least one fastener 1362 coupled thereto. This fastener 1362 may be the hook portion of a hook-and-loop fastener. Alternatively, as shown in FIGS. 21-22, the side panel 1304 may include fasteners 1320 similar to fasteners 120 as previously described with regard to FIG. 1.

As illustrated by FIG. 20, the side panel 1304 may be glued or bonded between an inner layer, or topsheet, 1330 and an outer layer, or backsheet, 1335 of the chassis 1302 in any manner that would be apparent to one of ordinary skill in the art.

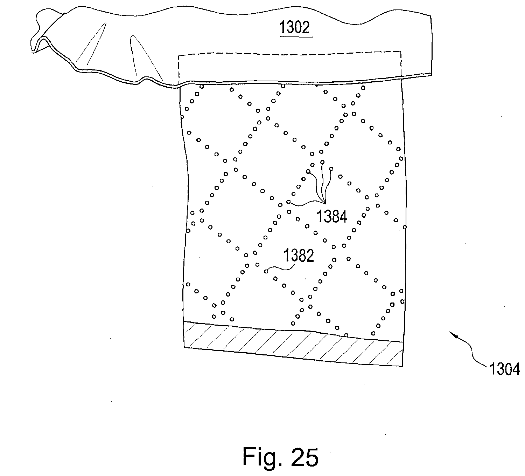

The first layer 1354, lamination layer 1350 and second layer 1352 may be bonded by the use of glue, heat and pressure bonding, heat bonding alone, pressure bonding alone, or ultrasonic bonding methods. Bonding patterns can be created in a continuous mode or a patterned, registered manner. For example, FIG. 25 shows a side panel 1304 for attachment to a chassis 1302 as illustrated in FIG. 17. The side panel includes bonding pattern 1382 that creates a quilted appearance and/or feel for the side panel. The bonding pattern may be created by bonding one or more of the layers at a number of different bonding sites 1384. Other bonding patterns may also be used. In addition, the edges of the first and second layers 1354, 1352 may be bonded or sealed along the periphery of the side panel.

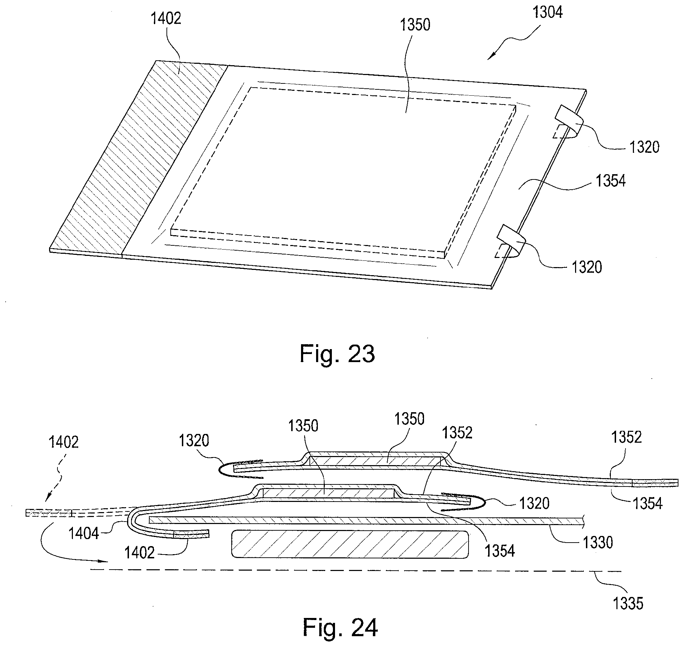

As illustrated by FIGS. 23-34, additional bonding 1402 may be performed after the time of laminating the lamination layer 1350 into the side panel 1304. This additional bonding can improve the process of applying and folding the product by virtue of creating a "fold line" 1404 in the side panel. This can be made to improve machine yield through reduced scrap, reduced inefficiency and reduction in out of spec products (folded side panels).

In an embodiment of the present invention, a skin care solution may be applied to the lamination layer 1350. The skin care solution may be absorbed by the lamination layer and then released onto the wearer's skin over a period of time. The skin care solution may be a lotion, gel, or other solution that could provide benefits to the wearer's skin, such as a reduction in irritation or ulcers. The skin care solution may include one or more ingredient known to promote healthy skin or prevent skin breakdown. Examples of such ingredients may include, but are not limited to, aloe vera, alpha hydroxy acids (including glycolic, lactic, malic, citric and tartaric acids), soy, grapeseed extract, vitamins A, C and E, coenzyme Q10, flavanoids, polyphenols, oat flour, honey, lactic acid, shea butter, petrolatum, lanolin, petrolatum, cyclomethicone, dimethicone copolyol, glyceryl stearates and propylene glycol linoleate, silicone, mineral oil, beeswax, caprylic/capric triglycerides, cyclomethicone, hydrogenated castor oil, mineral oil, lanolin, petroleum, petrolatum, silicone, dimethicone, vegetable oils, or any other ingredient know to improve skin condition. In further embodiments, a skin care solution may be applied to other absorbent portions of the absorbent article. For example, a skin care solution could be applied to one or more sections of the first absorbent core portion 116 or second absorbent core portion 118.

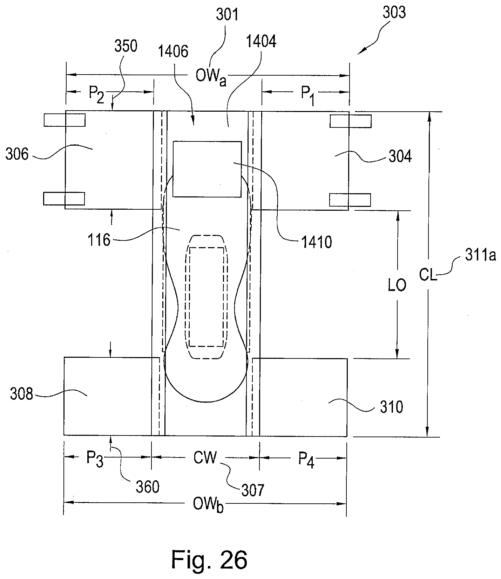

As illustrated in FIG. 26, a further embodiment of the invention that may include a sacral lamination layer 1410 may be added to the inside surface 1404 of a rear portion 1406 of the chassis 1302. The sacral lamination layer may be positioned such that it is positioned adjacent a users sacral region, in vicinity of the intergluteal cleft and the tailbone, when wearing the absorbent article. This lamination layer 1410 may overlap with one or more other components that make up the chassis 1302, such as the first core portion 116. The sacral lamination layer may include a high-loft material that is affixed to the chassis topsheet 1330, by adhesive or other bonding means know to one of skill in the art. A further embodiment may include a patient facing layer positioned over the sacral lamination layer. The high-loft material and patient facing layer may be similar to those described above for use in side panel 1304. A skin care solution, such as described above, may be applied to the sacral lamination layer 1410.

The sacral lamination layer 1410 may have a variety of different shapes, including rectangular, oval, or "butterfly" shaped. In addition, the sacral lamination layer or sacral lamination layer and patient facing layer may include one or more of a variety of different embossing shapes or patterns as described herein or as would be known to one skilled in the art. Embodiments of the invention contemplate absorbent articles using both a lamination layer 1350 in side panel 1304 and a sacral lamination layer 1410. Alternatively side panel lamination layer or sacral lamination layer may be used independently.

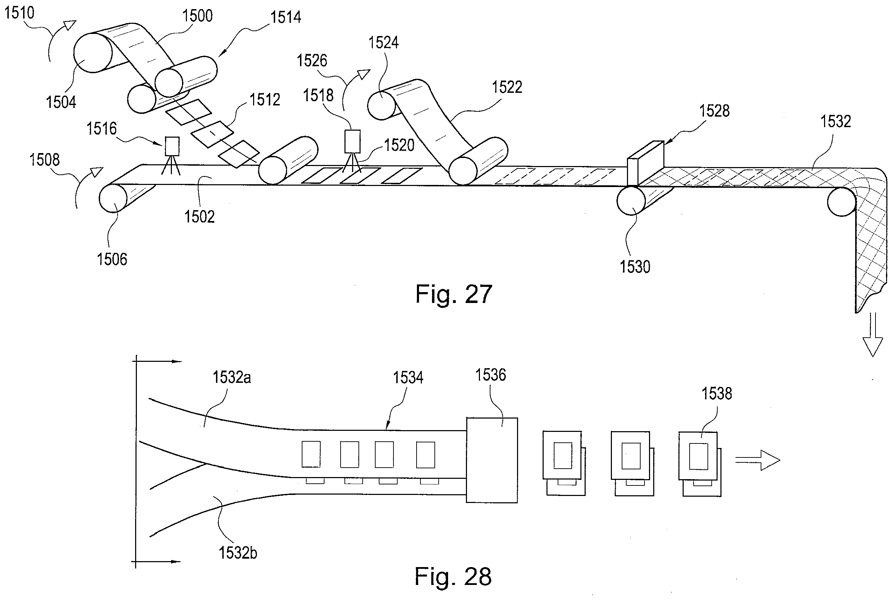

FIGS. 27-29 illustrate embodiments of a method for manufacturing an absorbent article with a rear side panel 1304 in accordance with embodiments shown in FIGS. 17-25. Embodiments of the manufacturing method may include the following steps: (1) Unwind a continuous web 1502 first facing layer 1354 of a side panel 1304 from a roll 1506 of facing material in a direction indicated by arrow 1508. (2) Unwind a continuous web 1500 of first lamination layer 1350 for the side panel from a roll 1504 of facing material in a direction indicated by arrow 1510. (3) Provide a short piece 1512 of lamination layer by feeding the lamination layer through a slip and cut 1514 (also known to those of skill in the art as cut and place) can also be performed with a cutting unit followed by a placing unit - as driven by either mechanical cam or servo motor unit, thereby providing an intermittent patch 1512 of lamination layer 1350 onto a continuous web of the first facing layer 1354 of the side panel 1304. This process may require adhesive 1516 to be applied to the first facing layer 1502. The slip/cut applicator may also require vacuum in order to hold the lamination layer within the unit until severed, accelerated and glued in place to the first facing layer. (4) Apply skin care lotion 1520 to the lamination layer piece 1512 through intermittent application using a spray applicator 1518, extrusion die, rotary gravure printing, or other applicator process. (5) Unwind a continuous web 1522 of second facing layer 1352 of side panel material from a roll 1524 of facing material in a direction indicated by arrow 1526. (6) Marry the first facing layer 1502 (with intermittent lamination layer patches 1512) with the second facing layer 1522. (7) Feed the combined layers through an ultrasonic bonding unit 1528, or a heated calendaring unit (also known as heat and pressure bonding unit), including a pattern roll 1530, to provide a quilted lamination pattern 1382 (or other, aesthetically pleasing pattern) which traps the lamination layer between the facing layers and provides an appearance of softness. (Hotmelt adhesive lamination can also be utilized for this application, but without benefit of a quilted bonding appearance) (8) Using the same pattern roll 1530, or a second means (which could be another ultrasonic bonding unit or a pressure unit, or a heated unit), provide a bonding pattern 1402 on the edges of the side panel 1304. (9) Add tape tabs 120 to the panel assemblies (not shown). An alternative to tape tabs as shown would be a continuous hook, a hook strip 1362 that is shorter than the panel assembly, or a plurality of hook strips that can be used to preferentially attach the side panel 1304 to either a corresponding side panel, or the front of the product's chassis 1302. Embodiments of the portion of the manufacturing process shown in FIG. 27 create a continuous panel web 1532.

FIG. 28 illustrates an embodiment of a further portion of the manufacturing process, including the following steps: (10) Provide a first continuous panel web 1532a and a second continuous panel web 1532b. The two panel webs 1532a, 1532b are overlapped in a slightly offset manner 1534. (11) A cutoff and spacing unit 1536 severs and spaces the panel webs into discrete pairs 1538 of side panels 1304.

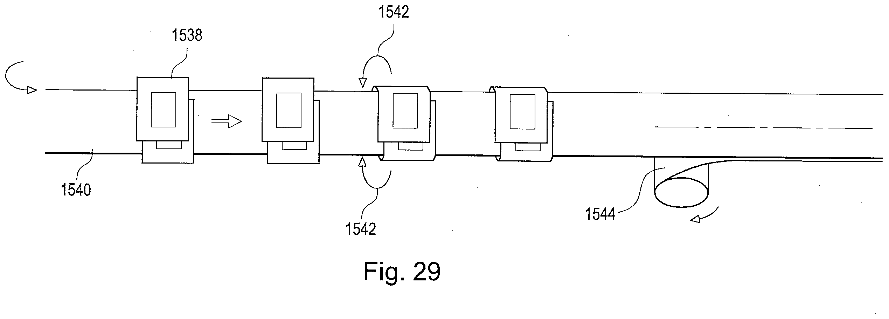

As illustrated in FIG. 29, an embodiment of a further portion of the manufacturing process includes the following steps: (12) Place the discrete pairs 1538 of side panels 1304 onto a continuous web 1540 of topsheet 1330 material. (13) Fold an edge of each side panel 1304 under the topsheet web 1540 along fold line 1404 as shown by arrows 1542. (14) Marry a continuous web 1544 of backsheet 1335 material, combined with absorbent cores, to the continuous web 1540 or the topsheet. (15) Adhere an edge of each side panel 1304 between the topsheet 1330 and the backsheet 1335, as illustrated in FIG. 20. In an alternate embodiment of this process, further side panels are manufactured without the tape tabs for use in a front side panel application.

While the present invention has been described with reference to one or more particular embodiments, those skilled in the art will recognize that many changes may be made thereto. Furthermore, components from one embodiment can be used in other non-exclusive embodiments. By way of example, any of the absorbent articles described herein can include any of the absorbent layers described in FIGS. 10-12. Each of these embodiments and obvious variations thereof is contemplated, which is set forth in the following alternative embodiments.

According to the present invention, an absorbent article is provided as disclosed in claim 1.

## Claims

1. An absorbent article, comprising:
a chassis (1302), the chassis (1302) including a length extending in a longitudinal direction from the back to the front of a user and a width extending in a lateral direction substantially perpendicular to the length between first and second longitudinal edges, the chassis (1302) comprising a first portion, a second portion and a crotch portion extending between the first and second portion, a portion of the chassis (1302) being configured to absorb fluids;
a first waist side panel (1304) operatively coupled to the first portion of the chassis (1302), the first waist side panel (1304) having a width extending in a lateral direction from the first longitudinal edge of the chassis first portion and a length extending longitudinally along the first longitudinal edge, the first waist side panel (1304) comprising:
a patient facing layer (1354);
an outwardly facing layer (1352); and
a lamination layer (1350) positioned between the patient facing layer (1354) and the outwardly facing layer (1352),
wherein the lamination layer (1350) has a higher loft than the patient facing layer (1354) or the outwardly facing layer (1352);
a second waist side panel operatively coupled to the second portion of the chassis (1302) in a position longitudinally spaced from the first waist side panel (1304) and configured to releasably attach to the first waist side panel (1304), the second waist side panel comprising:
a patient facing layer (1354);
an outwardly facing layer (1352); and
a lamination layer (1350) positioned between the patient facing layer (1354) and the outwardly facing layer (1352),
wherein the lamination layer (1350) has a higher loft than the patient facing layer (1354) or the outwardly facing layer (1352);
a third waist side panel operatively coupled to the first portion of the chassis (1302), the third waist side panel having a width extending in a lateral direction from the second longitudinal edge of the chassis first portion and a length extending longitudinally along the second longitudinal edge, the third waist side panel comprising:
a patient facing layer (1354);
an outwardly facing layer (1352); and
a lamination layer (1350) positioned between the patient facing layer (1354) and the outwardly facing layer (1352),
wherein the lamination layer (1350) has a higher loft than the patient facing layer (1354) or the outwardly facing layer (1352); and
a fourth waist side panel operatively coupled to the second portion of the chassis (1302) in a position longitudinally spaced from the third waist side panel and configured to releasably attach to the third waist side panel, the fourth waist side panel comprising:
a patient facing layer (1354);
an outwardly facing layer (1352); and
a lamination layer (1350) positioned between the patient facing layer and the outwardly facing layer,
wherein the lamination layer (1350) has a higher loft than the patient facing layer (1354) or the outwardly facing layer (1352).

2. The absorbent article of claim 1, wherein the lamination layer (1350) comprises a nonwoven material having a basis weight between 10 and 75 GSM.

3. The absorbent article of claim 1, wherein the lamination layer (1350) comprises a nonwoven material having a basis weight between 45 and 55 GSM.

4. The absorbent article of claim 1, wherein the patient facing layer (1354), the outwardly facing layer (1352) and the lamination layer (1350) are bonded at one or more bond sites (1384),
wherein the bond sites (1384) comprise at least one of an ultrasonic bond, a thermal bond and an adhesive bond.

5. The absorbent article of claim 4, wherein the one or more bond sites (1384) are arranged to form a bonding pattern (1382) that creates a quilted appearance for at least a portion of the side panel.

6. The absorbent article of claim 1, the patient facing layer (1354) having a width in a lateral direction extending parallel to the width of the waist side panel and a length extending longitudinally parallel to the length of the waist side panel; and
the lamination layer (1350) having a width in a lateral direction extending parallel to the width of the waist side panels and a length extending longitudinally parallel to the length of the waist side panels,
wherein, the width of the lamination layer (1350) is less than the width of the patient facing layer (1354), and
wherein the length of the lamination layer (1350) is less than the length of the patient facing layer (1354).

7. The absorbent article according to claim 1 or to any one of claims 4-6, wherein the lamination layer (1350) comprises:
an absorbent material; and
a skin care solution absorbed into the absorbent material.

8. The absorbent article of claim 7, wherein the skin care solution comprises a lotion.

9. The absorbent article according to claim 7 or 8, wherein the patient facing layer (1354) comprises a porous material configured such that the skin care solution transfers from the lamination layer (1350) through the patient facing layer (1354) to the skin of a user of the absorbent article.

10. The absorbent article according to any one of claims 7-9, wherein the lamination layer (1350) comprises at least one of a nonwoven material and a foam material.

11. The absorbent article according to claim 7,wherein the patient facing layer (1354), the outwardly facing layer (1352) and the lamination layer (1350) are bonded at one or more bond sites (1384), and
wherein the lamination layer (1350) perimeter is smaller than the patient facing layer (1354) perimeter and the outwardly facing layer (1352) perimeter, such that the waist side panels comprise a first portion having the patient facing layer (1354), the outwardly facing layer (1352), and the lamination layer (1350) and a second portion having the patient facing layer (1354) and the outwardly facing layer (1352) without the lamination layer (1350).

12. The absorbent article of claim 11, wherein at least one of the one or more bond sites (1384) is positioned on the first portion having the patient facing layer (1354), the outwardly facing layer (1352), and the lamination layer (1350).

13. The absorbent article of claim 11, wherein at least one of the one or more bond sites (1384) is positioned on the second portion having the patient facing layer (1354) and the outwardly facing layer (1352) without the lamination layer (1350).

14. The absorbent article according to any one of claims 1-13, wherein
the chassis comprises:
a topsheet;
a patient facing layer extending over a portion of the topsheet; and
a chassis lamination layer positioned between the topsheet and the patient facing layer, the chassis lamination layer comprising:
an absorbent material having a higher loft than the patient facing layer or the topsheet
a skin care solution absorbed into the absorbent material.

## Patentansprüche

1. Absorbierender Artikel mit:
einem Unterbau (1302), wobei der Unterbau (1302) eine Länge aufweist, die sich in einer Längsrichtung von der Rückseite zu der Vorderseite eines Benutzers erstreckt und eine Breite hat, die sich in einer Querrichtung im Wesentlichen senkrecht zu der Länge zwischen ersten und zweiten Längsrändern erstreckt, wobei der Unterbau (1302) einen ersten Bereich, einen zweiten Bereich und einen Zwickelbereich aufweist, der sich zwischen dem ersten und dem zweiten Bereich erstreckt, wobei ein Teil des Unterbaus (1302) dazu ausgebildet ist, Fluide zu absorbieren;
einem ersten Taillienseitenfeld (1304), das arbeitsmäßig mit dem ersten Bereich des Unterbaus (1302) gekoppelt ist, wobei das erste Taillienseitenfeld (1304) eine Breite hat, die sich in einer Querrichtung von dem ersten Längsrand des ersten Bereiches des Unterbaus (1302) erstreckt, und das eine Länge aufweist, die sich in Längsrichtung entlang des ersten Längsrandes erstreckt, wobei das erste Taillienseitenfeld (1304) Folgendes aufweist:
eine patientenseitige Schicht (1354);
eine auswärts gerichtete Schicht (1352); und
eine laminierte Schicht (1350), die zwischen der patientenseitigen Schicht (1354) und der auswärts gerichteten Schicht (1352) angeordnet ist,
wobei die laminierte Schicht (1350) einen höheren Boden als die patientenseitige Schicht (1354) oder die auswärts gerichtete Schicht (1352) hat;
ein zweites Taillienseitenfeld, das arbeitsmäßig mit dem zweiten Bereich des Unterbaus (1302) in einer Position gekoppelt ist, die in Längsrichtung von dem ersten Taillienseitenfeld (1304) beabstandet ist und das dazu ausgebildet ist, lösbar an dem ersten Taillienseitenfeld (1304) befestigt zu werden, wobei das zweite Taillienseitenfeld Folgendes aufweist:
eine patientenseitige Schicht (1354);
eine auswärts gerichtete Schicht (1352); und
eine laminierte Schicht (1350), die zwischen der patientenseitigen Schicht (1354) und der auswärts gerichteten Schicht (1352) angeordnet ist,
wobei die laminierte Schicht (1350) einen höheren Boden als die patientenseitige Schicht (1354) oder die auswärts gerichtete Schicht (1352) aufweist;
ein drittes Taillienseitenfeld, das arbeitsmäßig mit dem ersten Bereich des Unterbaus (1302) gekoppelt ist, wobei das dritte Taillienseitenfeld eine Breite hat, die sich in einer Querrichtung von dem zweiten Längsrand des Unterbaus des ersten Bereichs erstreckt und eine Länge aufweist, die sich in in Längsrichtung entlang des zweiten Längsrandes erstreckt, wobei das dritte Taillienseitenfeld Folgendes aufweist:
eine patientenseitige Schicht (1354);
eine auswärts gerichtete Schicht (1352); und
eine laminierte Schicht (1350), die zwischen der patientenseitigen Schicht (1354) und der auswärts gerichteten Schicht (1352) angeordnet ist,
wobei die laminierte Schicht (1350) einen höheren Boden als die patientenseitige Schicht (1354) oder die auswärts gerichtete Schicht (1352) aufweist; und
ein viertes Taillienseitenfeld, das arbeitsmäßig mit dem zweiten Bereich des Unterbaus (1302) in einer Position gekoppelt ist, die in Längsrichtung von dem dritten Taillienseitenfeld beabstandet ist und dazu ausgebildet ist, lösbar an dem dritten Taillienseitenfeld befestigt zu werden, wobei das vierte Taillienseitenfeld Folgendes aufweist:
eine patientenseitige Schicht (1354);
eine auswärts gerichtete Schicht (1352); und
eine laminierte Schicht (1350), die zwischen der patientenseitigen Schicht und der auswärts gerichteten Schicht angeordnet ist,
wobei die laminierte Schicht (1350) einen höheren Boden als die patientenseitige Schicht (1354) oder die auswärts gerichtete Schicht (1352) hat.

2. Absorbierender Artikel nach Anspruch 1, wobei die laminierte Schicht (1350) ein nicht gewebtes Material mit einem Basisgewicht zwischen 10 und 75 GSM aufweist.

3. Absorbierender Artikel nach Anspruch 1, bei dem die laminierte Schicht (1350) ein nicht gewebtes Material mit einem Basisgewicht zwischen 45 und 55 GSM aufweist.

4. Absorbierender Artikel nach Anspruch 1, bei dem die patientenseitige Schicht (1354), die auswärts gerichtete Schicht (1352) und die laminierte Schicht (1350) an einer oder mehreren Verbindungsstellen (1384) miteinander gebondet sind,
wobei die Verbindungsstellen (1384) wenigstens eine von einer Ultraschallverbindung, einer thermischen Verbindung und einer Klebeverbindung aufweisen.

5. Absorbierender Artikel nach Anspruch 4, bei dem die eine oder die mehreren Verbindungsstellen (1384) angeordnet sind, um ein Verbindungsmuster (1382) zu bilden, das wenigstens für einen Teil der Seitenfläche eine gesteppte Erscheinung bildet.

6. Absorbierender Artikel nach An- spruch 1, bei dem die patientenseitige Schicht (1354) eine Breite in einer Querrichtung aufweist, die sich parallel zu der Breite des Taillienseitenfeldes erstreckt, und eine Länge aufweist, die sich in Längsrichtung parallel zu der Länge des Taillienseitenfeldes erstreckt; und
wobei die laminierte Schicht (1350) eine Breite in einer Querrichtung aufweist, die sich parallel zu der Breite des Taillienseitenfeldes erstreckt und eine Länge aufweist, die sich in Längsrichtung parallel zu der Länge des Taillienseitenfeldes erstreckt,
wobei die Breite der laminierten Schicht (1350) geringer als die Breite der patientenseitigen Schicht (1354) ist, und
wobei die Länge der laminierten Schicht (1350) geringer als die Länge der patientenseitigen Schicht (1354) ist.

7. Absorbierender Artikel nach Anspruch 1 oder nach irgendeinem der Ansprüche 4 - 6, bei dem die laminierte Schicht (1350) Folgendes aufweist:
ein absorbierendes Material; und
eine Hautpflegelösung, die in dem absorbierenden Material absorbiert ist.

8. Absorbierender Artikel nach Anspruch 7, bei dem die Hautpflegelösung eine Lotion aufweist.

9. Absorbierender Artikel nach Anspruch 7 oder 8, bei dem die patientenseitige Schicht (1354) ein poröses Material aufweist, das derart ausgebildet ist, dass die Hautpflegelösung von der laminierter Schicht (1350) durch die patientenseitige Schicht (1354) zu der Haut eines Benutzers des absorbierenden Artikels gelangt.

10. Absorbierender Artikel nach irgendeinem der Ansprüche 7 - 9, bei dem die laminierte Schicht (1350) wenigstens eines von einem nicht gewebten Material und einem Schaummaterial aufweist.

11. Absorbierender Artikel nach Anspruch 7, bei dem die patientenseitige Schicht (1354), die auswärts gerichtete Schicht (1352) und die laminierte Schicht (1350) an einer oder mehreren Verbindungsstellen (1384) gebondet sind und
wobei die Umfangslänge der laminierten Schicht (1350) kleiner als die Umfangslänge der patientenseitigen Schicht (1354) und als die Umfangslänge der nach außen gerichteten Schicht (1352) ist, derart, dass die Taillienseitenfelder einen ersten Bereich aufweisen, der die patientenseitige Schicht (1354), die nach außen gerichtete Schicht (1352) und die laminierte Schicht (1350) hat, sowie einen zweiten Bereich, der die patientenseitige Schicht (1354) und die nach außen gerichtete Schicht (1352) ohne die laminierte Schicht (1350) hat.

12. Absorbierender Artikel nach Anspruch 11, bei dem wenigstens eine von der einen oder den mehreren Verbindungsstellen (1384) an dem ersten Bereich angeordnet ist, der die patientenseitige Schicht (1354), die nach außen gerichtete Schicht (1352) und die laminierte Schicht (1350) aufweist.

13. Absorbierender Artikel nach Anspruch 11, bei dem wenigstens eine oder mehrere der Verbindungsstellen (1384) an dem zweiten Bereich angeordnet ist, der die patientenseitige Schicht (1354) und die nach außen gerichtete Schicht (1352) ohne die laminierte Schicht (1350) aufweist.

14. Absorbierender Artikel nach irgendeinem der Ansprüche 1 - 13, bei dem der Unterbau Folgendes aufweist:
eine Decklage;
eine patientenseitige Schicht, die sich über einen Bereich der Deckschicht erstreckt; und
eine den Unterbau laminierende Schicht, die zwischen der Deckschicht und der patientenseitigen Schicht angeordnet ist, wobei die den Unterbau laminierende Schicht Folgendes aufweist:
ein absorbierendes Material, das einen höheren Boden als die patientenseitige Schicht oder die Deckschicht aufweist;
eine Hautpflegelösung, die in dem absorbierenden Material absorbiert ist.

## Revendications

1. Article absorbant, comprenant:
un châssis (1302), le châssis (1302) incluant une longueur s'étendant dans une direction longitudinale depuis dos jusqu'à l'avant d'un utilisateur et une largeur s'étendant dans une direction latérale sensiblement perpendiculaire à la longueur entre des premier et second bords longitudinaux, le châssis (1302) comprenant une première portion, une seconde portion et une portion d'entrejambe s'étendant entre la première et la seconde portion, une portion du châssis (1302) étant configurée pour absorber des fluides;
un premier panneau latéral de taille (1304) couplé opérationnellement à la première portion du châssis (1302), le premier panneau latéral de taille (1304) ayant une largeur s'étendant dans une direction latérale depuis le premier bord longitudinal de la première portion de châssis et une longueur s'étendant longitudinalement le long du premier bord longitudinal, le premier panneau latéral de taille (1304) comprenant:
une couche en regard du patient (1354);
une couche en regard vers l'extérieur (1352); et
une couche de stratification (1350) positionnée entre la couche en regard du patient (1354) et la couche en regard vers l'extérieur (1352),
dans lequel la couche de stratification (1350) a un gonflant supérieur à la couche en regard du patient (1354) ou à la couche en regard vers l'extérieur (1352);
un deuxième panneau latéral de taille couplé opérationnellement à la seconde portion du châssis (1302) dans une position espacée longitudinalement du premier panneau latéral de taille (1304) et configuré pour s'attacher de façon détachable au premier panneau latéral de taille (1304), le deuxième panneau latéral de taille comprenant:
une couche en regard du patient (1354);
une couche en regard vers l'extérieur (1352); et
une couche de stratification (1350) positionnée entre la couche en regard du patient (1354) et la couche en regard vers l'extérieur (1352),
dans lequel la couche de stratification (1350) a un gonflant supérieur à la couche en regard du patient (1354) ou à la couche en regard vers l'extérieur (1352);
un troisième panneau latéral de taille couplé opérationnellement à la première portion du châssis (1302), le troisième panneau latéral de taille ayant une largeur s'étendant dans une direction latérale depuis le second bord longitudinal de la première portion de châssis et une longueur s'étendant longitudinalement le long du second bord longitudinal, le troisième panneau latéral de taille comprenant :
une couche en regard du patient (1354);
une couche en regard vers l'extérieur (1352); et
une couche de stratification (1350) positionnée entre la couche en regard du patient (1354) et la couche en regard vers l'extérieur (1352),
dans lequel la couche de stratification (1350) a un gonflant supérieur à la couche en regard du patient (1354) ou à la couche en regard vers l'extérieur (1352); et
un quatrième panneau latéral de taille couplé opérationnellement à la seconde portion du châssis (1302) dans une position espacée longitudinalement du troisième panneau latéral de taille et configuré pour s'attacher de façon détachable au troisième panneau latéral de taille, le quatrième panneau latéral de taille comprenant:
une couche en regard du patient (1354);
une couche en regard vers l'extérieur (1352); et
une couche de stratification (1350) positionnée entre la couche en regard du patient (1354) et la couche en regard vers l'extérieur,
dans lequel la couche de stratification (1350) a un gonflant supérieur à la couche en regard du patient ou à la couche en regard vers l'extérieur (1352).

2. Article absorbant selon la revendication 1, dans lequel la couche de stratification (1350) comprend un matériau non tissé ayant une masse surfacique entre 10 et 75 g/m².

3. Article absorbant selon la revendication 1, dans lequel la couche de stratification (1350) comprend un matériau non tissé ayant une masse surfacique entre 45 et 55 g/m².

4. Article absorbant selon la revendication 1, dans lequel la couche en regard du patient (1354), la couche en regard vers l'extérieur (1352) et la couche de stratification (1350) sont liées au niveau d'un ou de plusieurs sites de liaison (1384),
dans lequel les sites de liaison (1384) comprennent au moins l'une d'une liaison ultrasonique, d'une liaison thermique et d'une liaison adhésive.

5. Article absorbant selon la revendication 4, dans lequel les un ou plusieurs sites de liaison (1384) sont agencés pour former un motif de liaison (1382) qui crée un aspect matelassé pour au moins une portion du panneau latéral.

6. Article absorbant selon la revendication 1, la couche en regard du patient (1354) ayant une largeur dans une direction latérale s'étendant parallèle à la largeur du panneau latéral de taille et une longueur s'étendant parallèle longitudinalement à la longueur du panneau latéral de taille; et
la couche de stratification (1350) ayant une largeur dans une direction latérale s'étendant parallèle à la largeur des panneaux de côté de taille et une longueur s'étendant parallèle longitudinalement à la longueur des panneaux de côté de taille,
dans lequel, la largeur de la couche de stratification (1350) est inférieure à la largeur de la couche en regard du patient (1354), et
dans lequel la longueur de la couche de stratification (1350) est inférieure à la longueur de la couche en regard du patient (1354).

7. Article absorbant selon la revendication 1 ou selon l'une quelconque des revendications 4 à 6, dans lequel la couche de stratification (1350) comprend :
un matériau absorbant; et
une solution de soin pour la peau absorbée dans le matériau absorbant.

8. Article absorbant selon la revendication 7, dans lequel la solution de soin pour la peau comprend une lotion.

9. Article absorbant selon la revendication 7 ou 8, dans lequel la couche en regard du patient (1354) comprend un matériau poreux configuré de sorte que la solution de soin pour la peau se transfère de la couche de stratification (1350) à travers la couche en regard du patient (1354) à la peau d'un utilisateur de l'article absorbant.

10. Article absorbant selon l'une quelconque des revendications 7 à 9, dans lequel la couche de stratification (1350) comprend au moins l'un d'un matériau non tissé et d'un matériau en mousse.

11. Article absorbant selon la revendication 7, dans lequel la couche en regard du patient (1354), la couche en regard vers l'extérieur (1352) et la couche de stratification (1350) sont liées au niveau d'un ou de plusieurs sites de liaison (1384), et
dans lequel le périmètre de la couche de stratification (1350) est plus petit que le périmètre de la couche en regard du patient (1354) et que le périmètre de la couche en regard vers l'extérieur (1352), de sorte que les panneaux de côté de taille comprennent une première portion ayant la couche en regard du patient (1354), la couche en regard vers l'extérieur (1352) et la couche de stratification (1350) et une seconde portion ayant la couche en regard du patient (1354) et la couche en regard vers l'extérieur (1352) sans la couche de stratification (1350).

12. Article absorbant selon la revendication 11, dans lequel au moins l'un des un ou plusieurs sites de liaison (1384) est positionné sur la première portion ayant la couche en regard du patient (1354), la couche en regard vers l'extérieur (1352) et la couche de stratification (1350).

13. Article absorbant selon la revendication 11, dans lequel au moins l'un des un ou plusieurs sites de liaison (1384) est positionné sur la seconde portion ayant la couche en regard du patient (1354) et la couche en regard vers l'extérieur (1352) sans la couche de stratification (1350).

14. Article absorbant selon l'une quelconque des revendications 1 à 13, dans lequel le châssis comprend:
une feuille de dessus;
une couche en regard du patient s'étendant sur une portion de la feuille de dessus; et
une couche de stratification de châssis positionnée entre la feuille de dessus et la couche en regard du patient, la couche de stratification de châssis comprenant:
un matériau absorbant ayant un gonflant supérieur à la couche en regard du patient ou à la feuille de dessus
une solution de soin pour la peau absorbée dans le matériau absorbant.
